# EUROPEAN PATENT APPLICATION

(11) **EP 1 410 762 A1**
(43) Date of publication of application: **21.04.2004**
(21) Application number: 03023006.4
(22) Date of filing: 11.10.2003
(51) Int. Cl.: A61B 17/15

(54) **System for orthopedic operations without additional invasivity**

(30) Priority: 18.10.2002 IT mi20022218
(71) Applicant: Medacta International S.A., 6874 Castel San Pietro (TI) (IT)
(72) Inventor: Bernardoni, Massimiliano, 8900 Lugano (CH); Furia, Giovanni, 6702 Claro (CH); Le Foll, Dominique, 95330 Donont (FR); Orlandini, Luca, 6900 Lugano (CH); Siccardi, Alberto, 6968 Sonvico (CH)
(74) Representative: Incollingo, Italo

(57) **Abstract**

System to carry out orthopedic chirurgical operations in particular for knee prosthesis, essentially involving an anchorage instrumentation at the distal end or epiphysis of the femur in order to reduce the patient's invasivity to that physiologically necessary for the knee total arthroplasty (TKA).

## Description

The present invention refers to a system for orthopedic operations, without additional invasivity over the minimal invasivity stricly requested by said operations.

In a very advantageous embodiment the system according to the invention assumes the form of an instrumentation (ancillary apparatus) for knee operations known as TKA (Total Knee Arthroplasty), which involve the articulation substitution with a prosthesis.

The present invention comprises also a method for the effective application of said instrumentation bridging the zone to be operated.

### Prior Art

The knee operations involve the articulation substitution by inserting a prosthesis generally comprising a femoral component (to be obviously associated to the femur end), a tibial component (at the tibia proximal end), and an intermediate component acting as an interface between said two components.

For the application of said components, cuts are needed on the limbs, in particular on the femoral and tibial epiphysis. At least one ancillary apparatus to guide the cut means (blades saws) is requested to be immovably associated to the limbs to be cut.

To this end a device of the femoral metaphysis has been proposed which comprises two autocentering jaws which are not applied to the epiphysis but to the femur metaphysis i.e. to a condyle posterior zone. To fix said jaws to the metaphysis, the conventional device shows pins or nails which penetrate, for some millimeters the cortical part by acting on the screw locking said jaws.

There is no doubt that this "pliers" device has merit however it is never free of inconveniences (both for the surgeon and the patient), among which we mention (without limitations): - the conventional "pliers" is very invasive above all because it insists on a proximal (or less distal) femur zone; - the patient's post-operation recovery is long and difficult also because a large portion of soft tissues is involved; - the fixing of said "pliers" on the metaphysis is per sé laborious and not safe in the frequent cases of bone sclerosis.

### Summary of the Invention

First object of the present invention is to provide an instrumentation which is free of the drawbacks of the Prior Art, especially free of the above mentioned inconveniences, and is easy to use, has a simple mechanical structure, is little invasive, easy to be applied and activate during the operations, and requires a very simple maintenance.

An other object of the invention is to provide a method for fixing and operating said auxiliary, which is particularly effective and reliable. Moreover the instrumentation and the relevant method according to the invention have the further advantage to be implemented on different limbs (for instance, left and right knee, tibial and femoral cuts etc.) and operable with the aid of various activating means in particular with robots.

The main characteristics of the invention are recited in the appended claims which are considered here incorporated.

### Brief Description of the Drawings

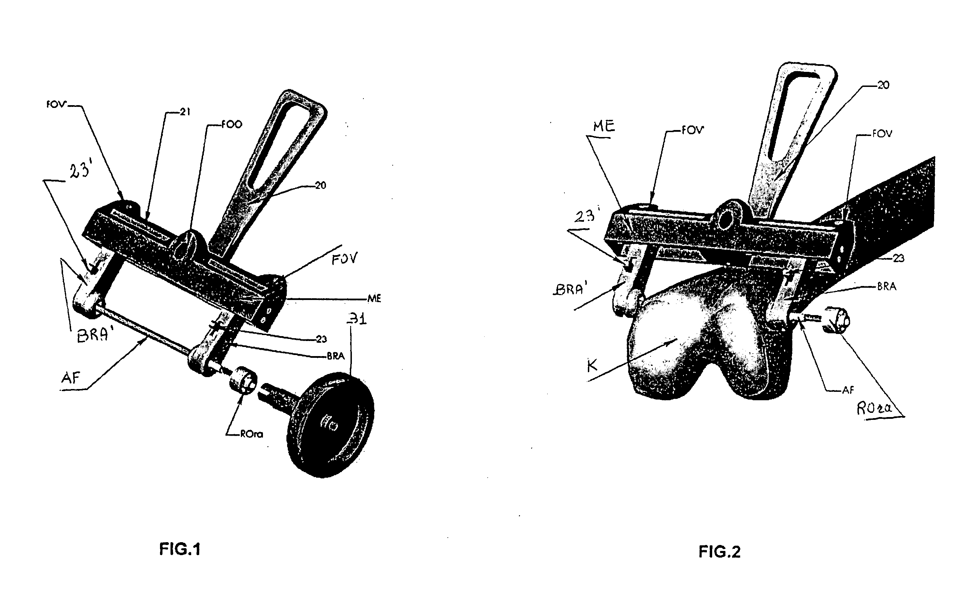

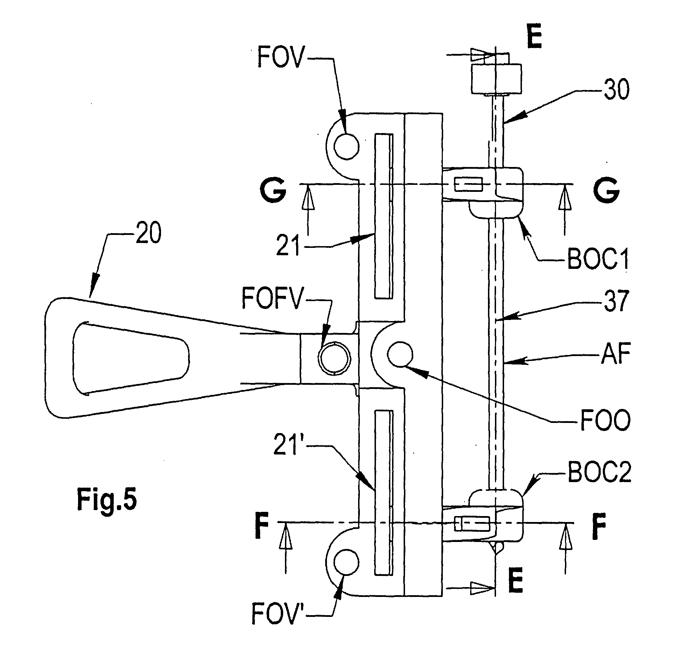

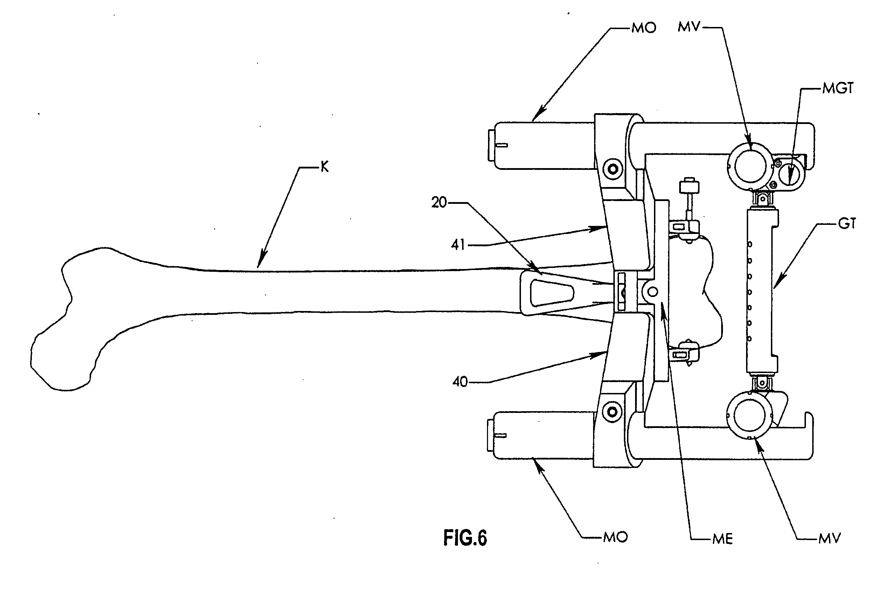

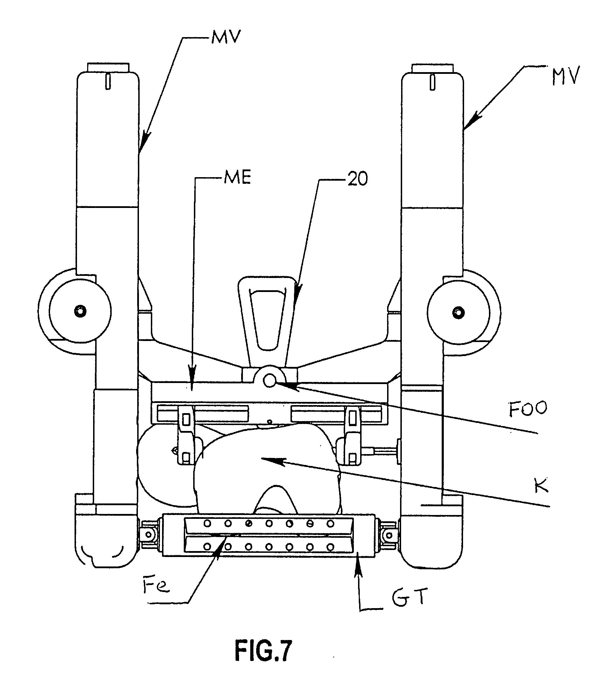

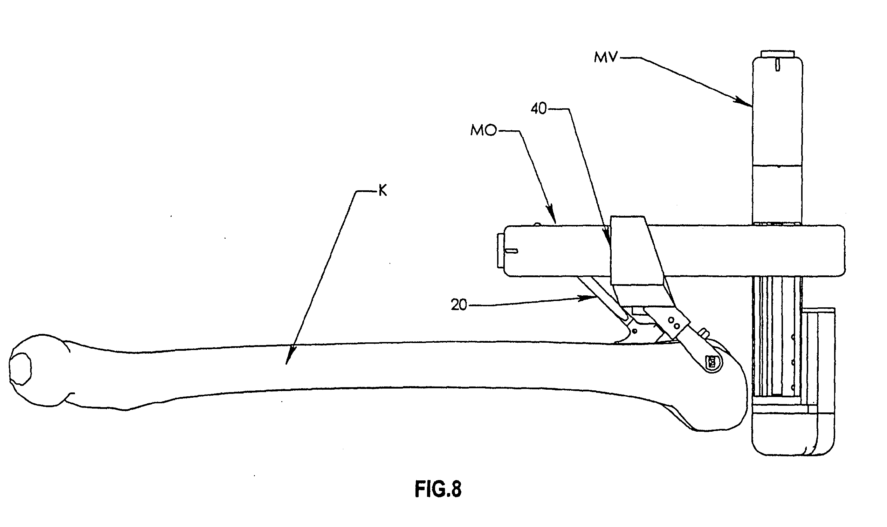

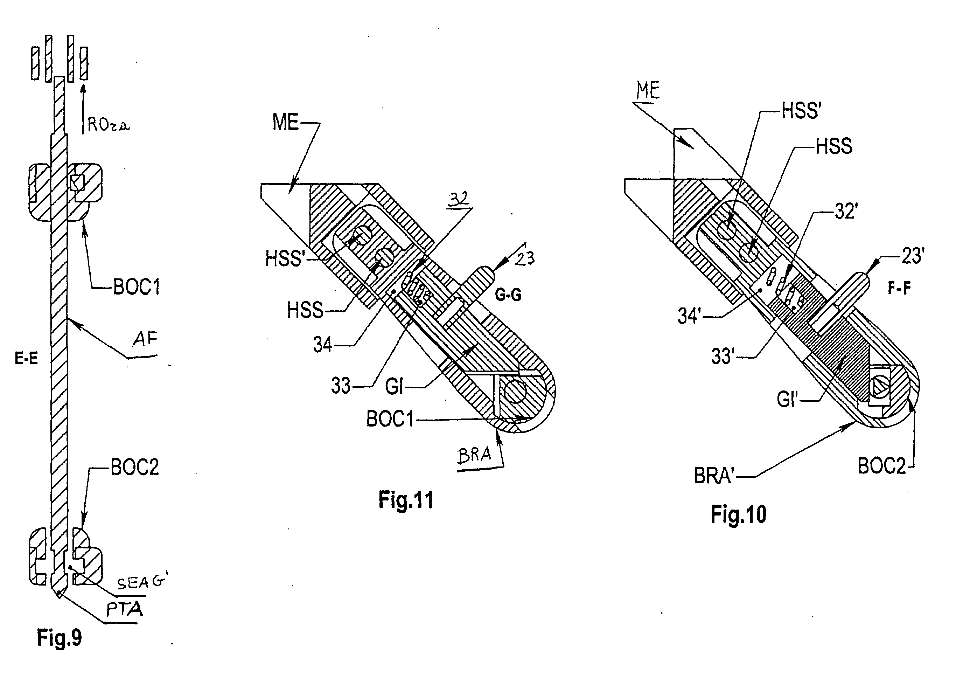

The various features and advantages of the invention will better appear from the following description of the (not limitative) embodiments shown in the accompanying drawings in which:
― Figures 1, 2, 3, 3' and 4 are schematic and partially perspectives views of the system according to the invention;
― Fig. 5 is a top view of the instrumentation;
― Figures 6, 7, and 8 are schematic front views of the ancillary apparatus of the invention which is applied on the femur of a flexed knee and is advantageously associated to a robot; and
― Figures 9, 10 and 11 are cross-sections with planes having the dashed lines respectively E-E on Fig. 5, F-F on Fig. 10 and G-G on Fig. 11.
In the above figures are indicated with: - K the knee (in particular the femur); - STRU the whole instrumentation or ancillary apparatus, which substantially comprises a stationary frame consisting of: - a small shovel or grip (preferably not removable) 20; - a bracket ME (substantially perpendicular to 20); - two arms BRA, BRA' parallel to 20 and perpendicular to ME; - the small levers 23 acting on the guillotines GI, GI' (figures 10, 11); - and AF the fixation rod of the instrumentation to the knee K.

In details, in the bracket ME are drawn: - at least a first hole FOO for the passage of a horizontal pin (not shown because it is generally inside the robot RO); - and at least a couple of vertical holes FOV, FOV' for the passage of at least two vertical pins (also not-shown because are inside the robot).

In the small shovel 20 which is utilized by the surgeon to grip the instrumentation STRU, is drawn at least one hole FOF.V for the passage of a screw (not shown) which insures the stability of the fixed arms BRA-BRA' of the bracket ME by means of bushes BOC1-BOC2 and of washer RO.ra.

This last washer is secured to the rod AF through a threading 30 (fig. 5); the locking of said washer RO.ra to the rod AF takes place by f.i. a handle 31 (fig. 1). At the not-threaded end of rod AF (opposite to the washer RO.ra) are drawn: - a pyramidal point PTA for the perforation of the femoral bone (in this case), and a triangular portion SEAGI for the engagement of the guillotine GI' (fig. 10) which slides by displacing the small levers 23, 23', and of the compression springs 32, 32' inserted in the "ad hoc" pits 33, 33', 34, 34'.

The adaptation of the system (instrumentation) STRU to the possible various sizes of knee K (in the case the femur) takes place by the horizontal sliding along the axis of the fixing rod AF, of the arms BRA and BRA' on the two small columns HSS and HSS', as better shown in fig. 12 in which are also represented the compression springs 38, 38' for the rapid unhook of the instrumentation STRU from femur F. Preferably the bracket ME is provided with slots 21, 21' for the maintenance and the washing of the ancillary apparatus STRU.

According to an advantageous feature of the invention the sliding of arms BRA-BRA' on the columns HSS-HSS' is strongly facilitated by a critical choice of the materials of said arms and small columns.

In preferred embodiment, the arms are made of steel AISI 420B and the columns of the hard sinterized metal known as HSS. The adoption of (at least) two (and not one) columns allows the compensation of the relevant clearance or end play between arms and columns preventing possible crowlings or blocks of said arms on said columns during the locking phase. The material choice is made to enhance the slidings and to (avoid possible seizures between arms and columns which seizures could produce scratches on the columns and shutdowns of the system.

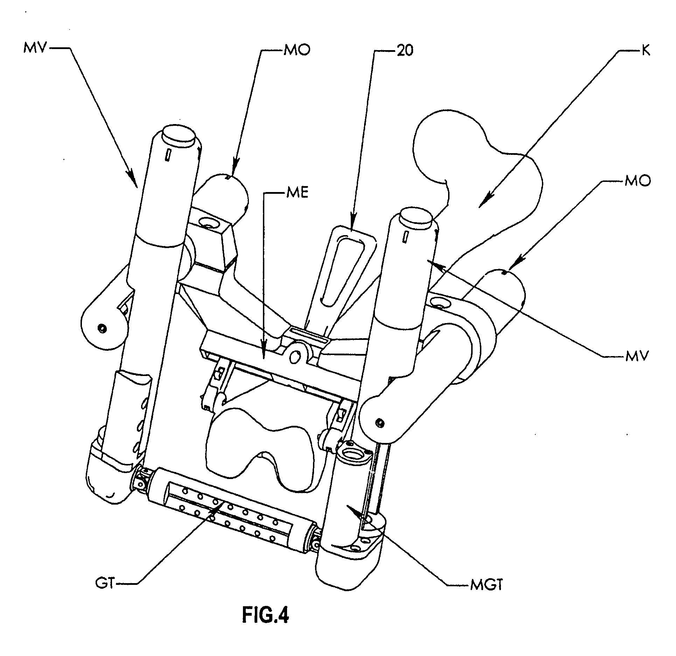

In the figures 4, 6, 7 and 8 is schematically represented a robot RO which is provided with suitable arms 40-41 to couple with instrumentation STRU; preferably the lower ends of said arms 40, 41 penetrate in at least the holes FOO-FOO' and FOV which are better substituted with a series of holes (or hole couples) lo allow movements of the robot and choice of its positions.

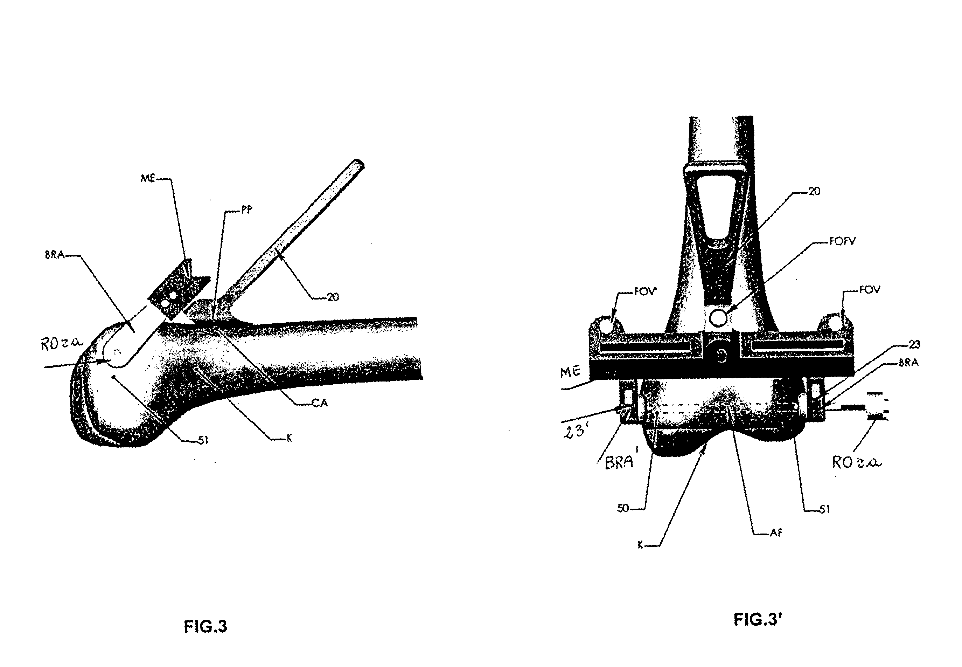

The method of using the instrumentation STRU, with or without robot, is articulated in the following phases:
I - the surgeon positions STRU on the preliminary exposed knee (figures 2, 3, 6, 7 and 8) by bringing (according to an advantageous feature of the invention) the posterior PP (fig. 3) of plate 20 to coincide with the anterior cortical CA at the level of the femoral epiphysis and no more, of the metaphysis which involved additional invasivity;
II - preferably the cortical screw VFC (figures 3, 3') is hooked by passing through the hole FO.FV and penetrating for, f.i. 10-30 mm in said anterior cortical zone CA; in an advantageous variant, instead of the cortical screw VFC (which requires and leaves big holes) small pins are inserted in oblique very small holes;
III - by manually maintaining arms BRA-BRA' in contact with the epicondyles 50-50', the whole rod AF (in dashed lines in fig. 3) is inserted with the aid f.i. of a drill, till the snap penetration of the guillotine GI (oe GI') in the "ad hoc" triangular seat SEAG' drawn on the rod AF;
IV - the washer RO.ra is activated f.i. by turning the manual grip 31 (fig. 1) till the locking of STRU on K with the maximal stability;
V - at the end of the operation the surgeon acts on the lever 23-23' and unhooks rapidly the arms which under the action of springs 38-38' widen allowing the extraction of rod AF on the washer side.

For illustrative clarity the invention has been described with particular reference to the embodiments represented in the accompanying drawings. It is obvious that all the changes, variants, substitutions, additions and the like to said embodiments, which are in the reach of a technician skilled in the art, are to be considered as falling within the scope and spirit of the invention in particular as recited in the following claims.

## Claims

1. System for carrying out chirurgical orthopedic operations, in particular of knee prosthesis, essentially comprising an instrumentation or ancillary apparatus for anchorage to the distal end or epiphysis of the femur so to reduce the patient's invasivity to that physiologically necessary for the knee total arthroplasty (TKA).

2. System according to claim 1, comprising a robot to enslave said anchorage.

3. System according to claim 1, in which said anchorage of the ancillary apparatus is embodied with a device at least comprising: - a bracket; - at least two arms perpendicular to the axis of said bracket; - at least two fixing bushes associated to said arms; - at least one intercondylar pin also of fixation; - and means for the stationary application of said instrumentation.

4. System according to claim 3, in which said bracket comprises at least one holding small plate having on its base at least a through hole to receive a cortical auto-threading screw; - at least three holes for the passage of the anchorage pins of the surgical cut-helping ancillary apparatus; - at least one pit to receive said arms and the relevant sliding columns; - and fensters for the maintenance and washing of the whole instrumentation.

5. System according to claims 2 and 4, in which said at least three holes are utilized by expansions (slots) drawn in the cut helping ancillary, particularly in form of a robot.

6. System according to at least one of the above claims, in which at least one of said bushes guides the bone perforation, and at least an other bush irremovably, locks the rod within the arm by means of end perforation bushes.

7. System according to at least on of the above claims, in which the guide bush shows a blind bottom slot for its retention within the arm by a guillotine whereas said block bush shows a passing slot which besides retaining itself inside the relevant arm by means of its guillotine, carries out a snap lock of the perforation at the end of its stroke.

8. System according to at least one of the preceeding claims, in which said rod or pin is provided with a threading for the coupling with a locking washer.

9. System according to at least one of the above claims, in which arms and columns have different hardness of materials, in particular, the columns are of hard sintered metal HSS and the arms are made of AISI 420B steel.

10. System according to at least one of the above claims, in which the instrumentation has structure and function substantially comparable to a so-called musket or spring catch with projecting arms carrying at the free ends an intercondylar penetration rod.

11. Method to carry out knee surgical operations by utilizing a instrumentation (STRU) according to the above claims, **characterized by** at least following steps:
I - after exposing the knee, the posterior portion of the instrumentation (STRU) plate (20) is placed on the anterior cortical, at the level of the femoral epiphysis;
II - a cortical screw or, equivalently, pins are passed in the plate hole or holes (FOEV) and are penetrated for a depth of about 10-30 mm in the anterior cortical zone;
III - by keeping manually the arms (BRA-BRA') in contact with the epicondyles (50-51) the whole pin or rod (AF) is inserted in the knee till the snap-penetration of the guillotine (GI or GI') in a seat (SEAG') made on said pin (AF);
IV - the washer (RO-ra) is activated till the reaching of the instrumentation (STRU) lock on the knee (K) with the maximal stability;
V - at the end of the operation, by acting on the smaller levers (23-23') the arms are rapidly released, which levers under the push of the springs (38-38') widen allowing the easy extraction of said pin or rod and consequently the removal of said instrumentation.
